# EUROPEAN PATENT APPLICATION

(11) **EP 4 831 635 A1**
(43) Date of publication of application: **28.10.2026**
(21) Application number: 24907513.6
(22) Date of filing: 19.12.2024
(51) Int. Cl.: G01N 33/543

(54) **AGENT FOR IMPROVING AGGLUTINATION REACTION, METHOD FOR IMPROVING TARGET ANTIGEN MEASUREMENT METHOD, AND REAGENT FOR MEASURING HUMAN BRAIN NATRIURETIC PEPTIDE**

(30) Priority: 22.12.2023 JP 2023216898
(71) Applicant: Denka Company Limited, Tokyo 103-8338 (JP)
(72) Inventor: IKENAGA, Yasuyuki, Tokyo 103-8338 (JP); KAJIWARA, Keigo, Tokyo 103-8338 (JP); WAKABAYASHI, Yuki, Tokyo 103-8338 (JP); YOSHIZAKI, Kyohei, Tokyo 103-8338 (JP)
(74) Representative: Hoffmann Eitle
(86) International application number: PCT/JP2024/045033
(87) International publication number: WO 2025/135129

(57) **Abstract**

An agent for improving an agglutination reaction is composed of a copolymer containing 2-methacryloyloxyethyl phosphorylcholine as a monomer unit. The agglutination reaction is an agglutination reaction of insoluble particles due to an antigen-antibody reaction between a target antigen and insoluble particles having a particulate carrier and an antibody against the target antigen supported on the particulate carrier, the antibody being supported on the particulate carrier. The agent is used to suppress a discrepancy in reactivity of the agglutination reaction between a system not including a blood sample and a system including a blood sample.

## Description

### TECHNICAL FIELD

The present invention relates to an agent for improving an agglutination reaction, a method for improving a target antigen measurement method, and a reagent for measuring human brain natriuretic peptide.

### BACKGROUND

As a method for measuring a target antigen utilizing an immunological reaction, there is a method utilizing particulate carriers such as latex (for example, Patent Document 1). Particulate carriers to which antibodies specific to a target antigen are bound cause an antigen-antibody reaction when the target antigen is present. Then, due to the specificity and affinity of the antibodies, the particulate carriers bind to one another via the target antigen and aggregate. The presence or absence and the amount of the target antigen are measured based on the presence or absence of the resulting aggregates and the degree of aggregation.

Such a measurement method utilizing an immunological reaction may have a problem in that detection sensitivity is insufficient, and for example, Patent Document 2 discloses that sensitivity of the measurement can be improved by carrying out the immunological reaction in the presence of polyvinylpyrrolidone.

### CITATION LIST

### PATENT LITERATURE

Patent Document 1: JP 2017-083440 A
Patent Document 2: JP H04-020859 A

### SUMMARY OF INVENTION

### TECHNICAL PROBLEM

A method for measuring a target antigen utilizing an immunological reaction as discussed above is applied to, for example, measurement of a target antigen contained in a specimen containing a blood sample. Because the reactivity of an agglutination reaction based on an immunological reaction is greatly affected by a blood sample, in order to accurately measure a target antigen, it is necessary to suppress a discrepancy in reactivity of the agglutination reaction between a system not including a blood sample and a system including a blood sample. However, when the present inventors examined the reactivity of the above-described agglutination reaction in the presence of polyvinylpyrrolidone, as described in Patent Document 2, the reactivity of the agglutination reaction in a system not including a blood sample and a system including a blood sample differed greatly.

In view of the above-described circumstances, an object of the present invention is to provide an agent that can sufficiently suppress a discrepancy in reactivity of the above-described agglutination reaction between a system not including a blood sample and a system including a blood sample, or a method for sufficiently suppressing such a discrepancy.

### SOLUTION TO PROBLEM

As a result of extensive research, the present inventors found that a copolymer containing 2-methacryloyloxyethyl phosphorylcholine as a monomer unit can sufficiently suppress a discrepancy in reactivity of an agglutination reaction due to an antigen-antibody reaction between a target antigen and insoluble particles having a particulate carrier and an antibody against the target antigen supported on the particulate carrier, the antibody being supported on the particulate carrier, the discrepancy occurring between a system not including a blood sample and a system including a blood sample. This led to the completion of the present invention.

The present invention, for example, provides the following inventions.
[1] An agent for improving an agglutination reaction, the agent being composed of a copolymer containing 2-methacryloyloxyethyl phosphorylcholine as a monomer unit,
   the agglutination reaction being an agglutination reaction of insoluble particles due to an antigen-antibody reaction between a target antigen and insoluble particles having a particulate carrier and an antibody against the target antigen supported on the particulate carrier, the antibody being supported on the particulate carrier,
   the agent being used to suppress a discrepancy in reactivity of the agglutination reaction between a system not including a blood sample and a system including a blood sample.
[2] The agent for improving an agglutination reaction according to [1], the copolymer being a copolymer containing a monomer unit having an anionic group.
[3] The agent for improving an agglutination reaction according to [1] or [2], the copolymer being a copolymer containing a monomer unit having a nonionic group.
[4] A method for suppressing a discrepancy between reactivity of an agglutination reaction in a system not including a blood sample and reactivity of an agglutination reaction in a system including a blood sample,
   in a method for measuring a target antigen, using insoluble particles having a particulate carrier and an antibody against the target antigen supported on the particulate carrier, the antibody being supported on the particulate carrier, based on an agglutination reaction of the insoluble particles due to an antigen-antibody reaction between the antibody and the target antigen,
   the agglutination reaction being carried out in the presence of a copolymer containing 2-methacryloyloxyethyl phosphorylcholine as a monomer unit.
[5] The method according to [4], the copolymer being a copolymer containing a monomer unit having an anionic group.
[6] The method according to [4] or [5], the copolymer being a copolymer containing a monomer unit having a nonionic group.
[7] A reagent for measuring human brain natriuretic peptide, the reagent containing a copolymer containing 2-methacryloyloxyethyl phosphorylcholine as a monomer unit, and
   the measurement, using insoluble particles having a particulate carrier and an anti-human brain natriuretic peptide antibody supported on the particulate carrier, is based on an agglutination reaction of the insoluble particles due to an antigen-antibody reaction between the anti-human brain natriuretic peptide antibody and the human brain natriuretic peptide.

### EFFECTS OF INVENTION

According to the present invention, it is possible to provide an agent capable of sufficiently suppressing a discrepancy in reactivity of an agglutination reaction due to an antigen-antibody reaction between a target antigen and insoluble particles having a particulate carrier and an antibody against the target antigen supported on the particulate carrier, the antibody being supported on the particulate carrier, the discrepancy occurring between a system not including a blood sample and a system including a blood sample, or a method capable of sufficiently suppressing such a discrepancy.

### BRIEF DESCRIPTION OF DRAWINGS

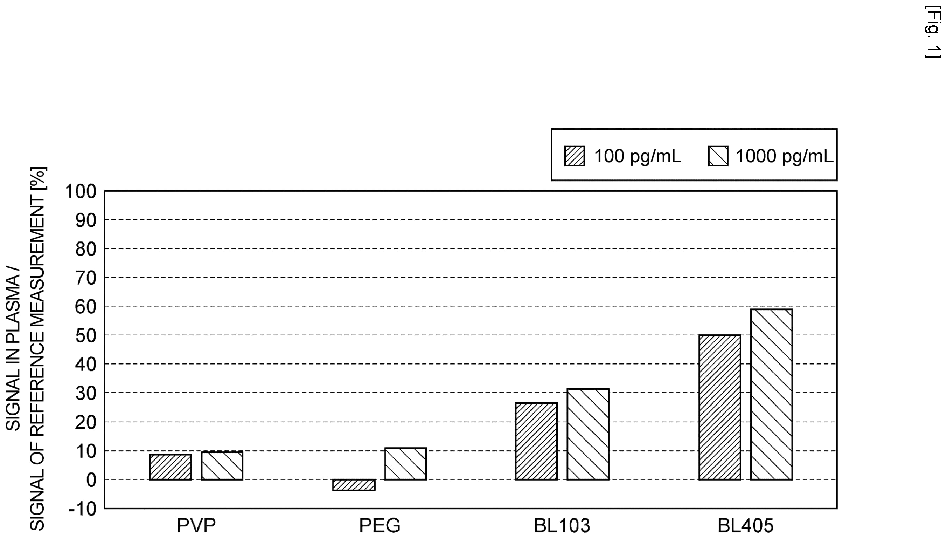

Fig. 1 is a graph showing a ratio of a change in absorbance in plasma to a change in absorbance due to an agglutination reaction of anti-human BNP antibody-sensitized latex in a 2 mass% BSA solution.

### DESCRIPTION OF EMBODIMENTS

Hereinafter, embodiments of the present disclosure will be described in detail.

An agent for improving an agglutination reaction according to one aspect of the present embodiment is composed of a copolymer (hereinafter also referred to as "MPC polymer") containing 2-methacryloyloxyethyl phosphorylcholine as a monomer unit, the agglutination reaction is an agglutination reaction of insoluble particles due to an antigen-antibody reaction between a target antigen and insoluble particles having a particulate carrier and an antibody against the target antigen supported on the particulate carrier, the antibody being supported on the particulate carrier (hereinafter also simply referred to as "agglutination reaction of insoluble particles"), and the agent is used to suppress a discrepancy in reactivity of the agglutination reaction between a system not including a blood sample and a system including a blood sample.

The reason why the MPC polymer suppresses a discrepancy in reactivity of an agglutination reaction of insoluble particles between a system not including a blood sample and a system including a blood sample is not necessarily clear. However, the present inventors consider that one factor is that, while a blood sample inhibits the agglutination reaction of insoluble particles, the MPC polymer suppresses such inhibition.

The reactivity of an agglutination reaction of insoluble particles is evaluated by measuring, in a system in which the agglutination reaction is carried out, a change (endpoint method) or a rate of change (rate method) in absorbance. For example, a suspension containing insoluble particles, a test sample containing a target antigen, and an MPC polymer are mixed and placed in a cell, and light in a visible to near-infrared region, typically 300 nm to 1000 nm, preferably 500 nm to 900 nm, is irradiated from outside the cell, and a change or a rate of change in absorbance after the mixing is measured. The time for measuring the change or rate of change in absorbance can be 1 minute to 30 minutes, preferably 1 minute to 10 minutes, but is not limited thereto. The temperature at which the above-described agglutination reaction after mixing is carried out can be 30 °C to 40 °C, and can also be 33 °C to 37 °C, but is not limited thereto. The evaluation of the reactivity of an agglutination reaction of insoluble particles may be specifically carried out by a method described in the examples described below.

The MPC polymer may be a copolymer containing monomer units having an anionic group, monomer units having a nonionic group, monomer units having a cationic group, and the like. From the viewpoint of further suppressing a discrepancy in reactivity of the agglutination reaction of insoluble particles between a system not including a blood sample and a system including a blood sample, the MPC polymer is preferably a copolymer containing monomer units having an anionic group and/or monomer units having a nonionic group, and more preferably a copolymer containing monomer units having an anionic group.

As the monomer units having an anionic group described above, from the viewpoint of further suppressing a discrepancy in reactivity of the agglutination reaction of insoluble particles between a system not including a blood sample and a system including a blood sample, a compound obtained by substituting a hydrogen atom of a carboxyl group of methacrylic acid with an anionic group is preferred. Examples of MPC polymers containing the above-described compound as a monomer unit include, for example, LIPIDURE^{®}-BL405 and the like of the LIPIDURE^{®}-BL400 series.

As the monomer units having a nonionic group described above, from the viewpoint of further suppressing a discrepancy in reactivity of the agglutination reaction of insoluble particles between a system not including a blood sample and a system including a blood sample, a compound obtained by substituting a hydrogen atom of a carboxyl group of methacrylic acid with a nonionic group is preferred. A nonionic group means a group having no cationic functional group or anionic functional group. A nonionic group may, for example, be a group including a hydrophilic group, a hydrophobic group, a hydrogen bond donor, and the like. Examples of MPC polymers having the above-described compound as a monomer unit include, for example, LIPIDURE^{®}-BL103 and the like of the LIPIDURE^{®}-BL100 series, the LIPIDURE^{®}-BL200 series, the LIPIDURE^{®}-BL700 series, the LIPIDURE^{®}-BL800 series, the LIPIDURE^{®}-BL1000 series, the LIPIDURE^{®}-BL1200 series, or the LIPIDURE^{®}-BL1300 series.

Examples of the above-described monomer units having a cationic group include a compound obtained by substituting a hydrogen atom of a carboxyl group of methacrylic acid with a cationic group. Examples of MPC polymers having the above-described compound as a monomer unit include, for example, LIPIDURE^{®}-BL502 and the like of the LIPIDURE^{®}-BL500 series.

The amount of the MPC polymer present in a system in which the agglutination reaction is carried out may be 0.005 mass% to 2 mass%, or may be 0.01 mass% to 1 mass%, based on the total amount of the system.

The MPC polymer is not particularly limited so long as the MPC polymer can sufficiently suppress a discrepancy in reactivity of the agglutination reaction of insoluble particles, and may be added to a system in which the agglutination reaction is carried out in combination, as necessary, with a stabilizer such as bovine serum albumin, an inorganic salt such as calcium chloride, a preservative such as ProClin^{®} 300, a buffer such as Bicine (2-(bis(2-hydroxyethyl)amino)acetic acid) buffer solution, and the like.

The MPC polymer is not particularly limited, and may be one produced by a known method, or may be a commercially available product.

Examples of the blood sample include, for example, human-derived body fluids such as a plasma sample, a whole blood sample, and a serum sample, and dilutions thereof. A human-derived plasma sample or a dilution thereof is preferable.

Examples of the target antigen include, but are not limited to: protein markers such as C-reactive protein (CRP), prostate-specific antigen, ferritin, β-2 microglobulin, myoglobin, hemoglobin, albumin, and creatinine; immunoglobulins such as IgG, IgE, IgA, and IgM; various tumor markers; lipoproteins such as LDL, HDL, and TG; viral antigens such as influenza A virus, influenza B virus, RS virus (RSV), rhinovirus, rotavirus, norovirus, adenovirus, astrovirus, HAV, HBs, HCV, HIV, and EBV; bacterial antigens such as *Chlamydia trachomatis*, streptococci, *Bordetella pertussis*, *Helicobacter pylori*, *Leptospira*, *Treponema pallidum*, *Toxoplasma gondii*, *Borrelia*, *Legionella* bacteria, *Bacillus anthracis*, and MRSA; toxins produced by bacteria and the like; mycoplasma lipid antigens; peptide hormones such as human brain natriuretic peptide (hBNP) and human chorionic gonadotropin; steroids such as steroid hormones; physiologically active amines such as epinephrine and morphine; vitamins such as vitamin B group vitamins; prostaglandins; antibiotics such as tetracyclines; agrochemicals; and environmental hormones.

Examples of the particulate carrier include particles such as latex particles, ceramic particles, alumina particles, silica-alumina particles, and carbon black particles. Among these particles, latex particles are preferred. Examples of latex materials include polystyrene and divinylbenzene, and polystyrene is preferred. The average particle diameter of the particulate carrier can be 0.1 µm to 5 µm. The particle diameter of the particulate carrier can be measured by a dynamic light scattering method. Herein, "average particle diameter" refers to a particle diameter (median diameter) at which a cumulative value obtained from smaller particle diameters reaches 50% of the total in a volume-based particle diameter distribution curve obtained by a dynamic light scattering method.

Antibodies are not particularly limited so long as they can specifically bind to an antigen, and examples thereof include IgG, IgM, IgA, IgD, and IgE, with IgG being preferred. IgG is composed of two heavy chains (H chains) and two light chains (L chains). Each heavy chain is composed, in order from the N-terminus, of a variable region (VH), a first constant region (CH1), a second constant region (CH2), and a third constant region (CH3). Each light chain is composed, in order from the N-terminus, of a variable region (VL) and a constant region (CL). A portion composed of CH2 and CH3 is an Fc region. One heavy chain and one light chain are linked to each other by a disulfide bond between a cysteine residue present in CH1 and a cysteine residue present in CL. In addition, the heavy chains are linked to each other by disulfide bonds between cysteine residues present in a hinge region located between CH1 and CH2. Antibodies may be IgG fragments in which heavy chains are present, rIgG (reduced IgG) composed of one heavy chain and one light chain, fragments composed of two heavy chains, or fragments composed of one heavy chain.

The particulate carrier and the antibody can be bound to each other using conventional methods, such as a physical adsorption method or a chemical bonding method.

A method according to another aspect of the present embodiment is a method for suppressing a discrepancy between reactivity of an agglutination reaction in a system not including a blood sample and reactivity of an agglutination reaction in a system including a blood sample, in a method for measuring a target antigen, using insoluble particles having a particulate carrier and an antibody against the target antigen supported on the particulate carrier, the antibody being supported on the particulate carrier, based on an agglutination reaction of the insoluble particles due to an antigen-antibody reaction between the antibody and the target antigen, the agglutination reaction being carried out in the presence of an MPC polymer.

An example of the above-described measurement method is as follows. First, a plurality of standard samples containing a target antigen to be measured at various known concentrations are prepared, and for each of the standard samples, a change or rate of change in absorbance is measured by the method discussed above. Examples of the standard sample include a 0.1 mass% to 5 mass% bovine serum albumin solution containing the above-described target antigen. A calibration curve is drawn by plotting the concentration of the antigen to be measured in the standard samples on the horizontal axis and the measured change or rate of change in absorbance on the vertical axis. For an unknown test sample as well, a change or rate of change in absorbance is measured by the same method, and by fitting the measurement results to the above-described calibration curve, the target antigen in the test sample can be quantitatively evaluated. In addition, a threshold for the change or rate of change in absorbance can be set in advance, and when the threshold is exceeded, the presence of the target antigen in the test sample can be qualitatively evaluated.

A reagent for measuring hBNP according to yet another aspect of the present embodiment contains a copolymer containing 2-methacryloyloxyethyl phosphorylcholine as a monomer unit, and the above-described measurement, using insoluble particles having a particulate carrier and an anti-hBNP antibody supported on the particulate carrier, is based on an agglutination reaction of the insoluble particles due to an antigen-antibody reaction between the anti-hBNP antibody and the hBNP.

In measurement of hBNP using the above-described reagent, a discrepancy between reactivity in an agglutination reaction in a system not including a blood sample and reactivity in an agglutination reaction in a system including a blood sample is suppressed.

A method for measuring hBNP using the above-described reagent may be the same as the measurement method in the method according to another aspect of the present embodiment discussed above.

The reagent may contain insoluble particles having a particulate carrier and an anti-hBNP antibody supported on the particulate carrier.

### EXAMPLES

Examples are given below to describe the present invention in more detail. However, the present invention is not limited only to these examples.

### <Preparation of Stabilizing Solution>

A stabilizing solution was prepared by adding polyvinylpyrrolidone (hereinafter also referred to as "PVP") such that the concentration was 1 mass%, polyethylene glycol 20000 (hereinafter also referred to as "PEG") such that the concentration was 2 mass%, LIPIDURE^{®}-BL103 (manufactured by NOF CORPORATION; hereinafter also referred to as "BL103") such that the concentration was 1.5 mass%, or LIPIDURE^{®}-BL405 (manufactured by NOF CORPORATION; hereinafter also referred to as "BL405") such that the concentration was 0.5 mass%, to a 120 mM Bicine (2-(bis(2-hydroxyethyl)amino)acetic acid) buffer solution containing 300 mM potassium chloride, 1 mass% bovine serum albumin (hereinafter also referred to as "BSA"), and 0.05 mass% ProClin^{®} 300.

### <Preparation of Antibody-Immobilized Latex Dispersion>

A solution of an anti-human brain natriuretic peptide (hereinafter referred to as "hBNP") antibody, the antibody being a mouse-derived IgG1 antibody, and polystyrene beads (average particle diameter: 0.3 µm) were mixed and allowed to react at 25 °C for 1 hour. Thereafter, centrifugation was performed, the supernatant was removed, and a 1 mass% BSA solution was added for blocking to obtain an antibody-immobilized latex. The obtained antibody-immobilized latex was adjusted by adding a dispersion medium such that the final concentration of the obtained antibody-immobilized latex was 0.125 mass%, thereby obtaining an antibody-immobilized latex dispersion. As the above-described dispersion medium, a dispersion medium containing 5 mM 3-morpholinopropanesulfonic acid (pH 7.0), 5 mass% sucrose, and 0.4 mass% BSA was used.

### <Evaluation of Reactivity of Agglutination Reaction>

Using a two-reagent-type latex agglutination immunoassay that uses a stabilizing solution and an antibody-immobilized latex dispersion, the effects of LIPIDURE^{®}-BL103 and LIPIDURE^{®}-BL405 on the discrepancy between reactivity of an agglutination reaction in a 2 mass% BSA solution and reactivity of an agglutination reaction in plasma were evaluated. Specifically, first, an hBNP solution was added to a 2 mass% BSA solution and human plasma such that the hBNP content therein was 0 pg/ml, 100 pg/ml, or 1000 pg/ml, thereby preparing hBNP-containing samples. The amount of the hBNP solution introduced in the above-described hBNP-containing samples was 10 mass% based on the total amount of the hBNP-containing samples. To 10 µL of the obtained hBNP-containing samples, 33 µL of the above-described stabilizing solution was added and mixed, and then the mixture was allowed to react at 37 °C for 5 minutes. Thereafter, 100 µL of the above-described antibody-immobilized latex dispersion was added and, after stirring, the mixture was allowed to react at 37 °C for 5 minutes. The absorbance over the 5-minute period after addition of the antibody-immobilized latex dispersion was continuously measured using a clinical chemistry analyzer TBA-120FR, with photometric points set at 21-23 and 31-33 and the main measurement wavelength set to 628 nm, and the change in absorbance during the 5-minute period was calculated. Next, for each stabilizing solution, the percentage of the above-described change in absorbance in human plasma containing hBNP at 100 pg/ml or 1000 pg/ml relative to the above-described change in absorbance in a 2 mass% BSA solution containing hBNP at the same concentration was calculated, and the results are shown in Figure 1. In Fig. 1, "SIGNAL IN PLASMA / SIGNAL OF REFERENCE MEASUREMENT [%]" indicates the above-described percentage.

As shown in Fig. 1, when a stabilizing solution containing Lipidure^{®}-BL103 or Lipidure^{®}-BL405 was used, the discrepancy between reactivity of the agglutination reaction in a 2 mass% BSA solution and reactivity of the agglutination reaction in human plasma was suppressed as compared to when a stabilizing solution containing polyvinylpyrrolidone or polyethylene glycol 20000 was used. In addition, this improvement effect was more pronounced in the stabilizing solution containing LIPIDURE^{®}-BL405.

From the above, it was shown that the use of a copolymer containing 2-methacryloyloxyethyl phosphorylcholine as a monomer unit makes it possible to sufficiently suppress the discrepancy between reactivity of the agglutination reaction in a system not including a blood sample and reactivity of the agglutination reaction in a system including a blood sample.

## Claims

1. An agent for improving an agglutination reaction, the agent being composed of a copolymer comprising 2-methacryloyloxyethyl phosphorylcholine as a monomer unit, wherein:
the agglutination reaction is an agglutination reaction of insoluble particles due to an antigen-antibody reaction between a target antigen and insoluble particles having a particulate carrier and an antibody against the target antigen supported on the particulate carrier; the antibody being supported on the particulate carrier, and
the agent is used to suppress a discrepancy in reactivity of the agglutination reaction between a system not including a blood sample and a system including a blood sample.

2. The agent for improving an agglutination reaction according to claim 1, wherein the copolymer is a copolymer comprising a monomer unit having an anionic group.

3. The agent for improving an agglutination reaction according to claim 1 or 2, wherein the copolymer is a copolymer comprising a monomer unit having a nonionic group.

4. A method for suppressing a discrepancy between reactivity of an agglutination reaction in a system not including a blood sample and reactivity of an agglutination reaction in a system including a blood sample,
in a method for measuring a target antigen, using insoluble particles having a particulate carrier and an antibody against the target antigen supported on the particulate carrier, the antibody being supported on the particulate carrier, based on an agglutination reaction of the insoluble particles due to an antigen-antibody reaction between the antibody and the target antigen,
wherein the agglutination reaction is carried out in the presence of a copolymer comprising 2-methacryloyloxyethyl phosphorylcholine as a monomer unit.

5. The method according to claim 4, wherein the copolymer is a copolymer comprising a monomer unit having an anionic group.

6. The method according to claim 4 or 5, wherein the copolymer is a copolymer comprising a monomer unit having a nonionic group.

7. A reagent for measuring human brain natriuretic peptide, the reagent comprising a copolymer comprising 2-methacryloyloxyethyl phosphorylcholine as a monomer unit, wherein:
the measurement, using insoluble particles having a particulate carrier and an anti-human brain natriuretic peptide antibody supported on the particulate carrier, is based on an agglutination reaction of the insoluble particles due to an antigen-antibody reaction between the anti-human brain natriuretic peptide antibody and the human brain natriuretic peptide.
